# EUROPEAN PATENT APPLICATION

(11) **EP 1 834 649 A1**
(43) Date of publication of application: **19.09.2007**
(21) Application number: 06005129.9
(22) Date of filing: 14.03.2006
(51) Int. Cl.: A61K 39/35

(54) **Method of developing a process for producing an allergen extract**

(71) Applicant: Alk-Abelló A/S, 2970 Hørsholm (DK)
(72) Inventor: Jacobi, Henrik Hugo, 2950 Vedbæk (DK); Beck, Tine Charlotte, 3460 Birkerød (DK); Ipsen, Hans-Henrik, 3400 Hillerød (DK); Stavnsbjerg, Merete, 3450 Allerød (DK)
(74) Representative: Inspicos A/S

(57) **Abstract**

The present invention relates to a method of developing a process for producing an allergen extract from a biological allergen source material comprising the steps of
a) subjecting the source material to a first extraction and purification process to produce a first purified allergen extract,
b) subjecting the first purified allergen extract to one or more toxicity tests to examine the first purified allergen extract for a potential content of one or more toxic components,
c) if the toxicity test shows that the toxic component has not been removed, subjecting the allergen extract to a first modified extraction and purification method to produce a first modified purified allergen extract,
d) subjecting the first modified purified allergen extract to one or more toxicity tests to examine the first modified purified allergen extract for a potential content of one or more toxic components,
e) repeating step c) to d) until the toxic component has been removed to identify an extraction and purification method, which removes the potential toxic component.

## Description

### TECHNICAL FIELD

The present invention relates to a method of developing a process for producing an allergen extract from a biological allergen source material. It also relates to a method of developing a process for producing an allergen composition from a gene expression medium mixture.

### BACKGROUND OF THE INVENTION

Allergy is a major health problem in countries where Western lifestyle is adapted. Furthermore, the prevalence of allergic disease is increasing in these countries. Although allergy in general may not be considered a life-threatening disease, asthma annually causes a significant number of deaths. An exceptional prevalence of about 30% in teenagers conveys a substantial loss in quality of life, working days and money, and warrants a classification among major health problems in the Western world.

Allergy is a complex disease. Many factors contribute to the sensitisation event. Among these is the susceptibility of the individual defined by an as yet insufficiently understood interplay between several genes. Another important factor is allergen exposure above certain thresholds. Several environmental factors may be important in the sensitisation process including pollution, childhood infections, parasite infections, intestinal microorganisms, etc. Once an individual is sensitised and the allergic immune response established, the presence of only minute amounts of allergen is efficiently translated into symptoms.

The natural course of allergic disease is usually accompanied by aggravation at two levels. Firstly, a progression of symptoms and disease severity, as well as disease progression, for example from hay fever to asthma. Secondly, dissemination in offending allergens most often occurs resulting in allergic multi-reactivity. Chronic inflammation leads to a general weakening of the mucosal defense mechanisms resulting in unspecific irritation and eventually destruction of the mucosal tissue. Infants may become sensitised primarily to foods, i.e. milk, resulting in eczema or gastrointestinal disorders; however, most often they outgrow these symptoms spontaneously. These infants are at risk of developing inhalation allergy later in their lives.

The most important allergen source are found among the most prevalent particles of a certain size in the air we breathe. These sources are remarkably universal and include grass pollens and house dust mite faecal particles, which together are responsible for approximately 50% of all allergies. Of global importance are also animal dander, i.e. cat and dog dander, other pollens, such as mugwort pollens, and micro-fungi, such as Alternaria. On a regional basis yet other pollens may dominate, such as birch pollen in Northern and Central Europe, ragweed in the Eastern and Central United States, and Japanese cedar pollen in Japan. Insects, i.e. bee and wasp venoms, and foods each account for approximately 2% of all allergies.

Allergy, i.e. type I hyper-sensitivity, is caused by an inappropriate immunological reaction to foreign non-pathogenic substances. Important clinical manifestations of allergy include asthma, hay fever, eczema, and gastro intestinal disorders. The allergic reaction is prompt and peaks within 20 minutes upon contact with the offending allergen. Furthermore, the allergic reaction is specific in the sense that a particular individual is sensitised to particular allergen(s), whereas the individual does not necessarily show an allergic reaction to other substances known to cause allergic disease. The allergic phenotype is characterized by a pronounced inflammation of the mucosa of the target organ and by the presence of allergen specific antibody of the IgE class in the circulation and on the surfaced of mast-cells and basophils.

An allergic attack is initiated by the reaction of the foreign allergen with allergen specific IgE antibodies, when the antibodies are bound to high affinity IgE specific receptors on the surface of mast-cells and basophils. The mast-cells and basophils contain preformed mediators, i.e. histamine, tryptase, and other substances, which are released upon cross-linking of two or more receptor-bound IgE antibodies. IgE antibodies are cross-linked by the simultaneous binding of one allergen molecule. It therefore follows that a foreign substance having only one antibody binding epitope does not initiate an allergic reaction. The cross-linking of receptor bound IgE on the surface of mast-cells also leads to release of signaling molecules responsible for the attraction of eosinophils, allergen specific T-cells, and other types of cells to the site of the allergic response. These cells in interplay with allergen, IgE and effector cells, lead to a renewed flash of symptoms occurring 12-24 hours after allergen encounter (late phase reaction).

Allergy disease management comprises diagnosis and treatment including prophylactic treatments. Diagnosis of allergy is concerned with by the demonstration of allergen specific IgE and identification of the allergen source. In many cases a careful anamnesis may be sufficient for the diagnosis of allergy and for the identification of the offending allergen source material. Most often, however, the diagnosis is supported by objective measures, such as skin prick test, blood test, or provocation test.

The therapeutic options fall in three major categories. The first opportunity is allergen avoidance or reduction of the exposure. Whereas allergen avoidance is obvious e.g. in the case of food allergens, it may be difficult or expensive, as for house dust mite allergens, or it may be impossible, as for pollen allergens. The second and most widely used therapeutic option is the prescription of classical symptomatic drugs like anti-histamines and steroids. Symptomatic drugs are safe and efficient; however, they do not alter the natural cause of the disease, neither do they control the disease dissemination. The third therapeutic alternative is specific allergy vaccination that in most cases reduces or alleviates the allergic symptoms caused by the allergen in question.

Conventional specific allergy vaccination is a causal treatment for allergic disease. It interferes with basic immunological mechanisms resulting in persistent improvement of the patients' immune status. Thus, the protective effect of specific allergy vaccination extends beyond the treatment period in contrast to symptomatic drug treatment. Some patients receiving the treatment are cured, and in addition, most patients experience a relief in disease severity and symptoms experienced, or at least an arrest in disease aggravation. Thus, specific allergy vaccination has preventive effects reducing the risk of hay fever developing into asthma, and reducing the risk of developing new sensitivities.

The immunological mechanism underlying successful allergy vaccination is not known in detail. A specific immune response, such as the production of antibodies against a particular pathogen, is known as an adaptive immune response. This response can be distinguished from the innate immune response, which is an unspecific reaction towards pathogens. An allergy vaccine is bound to address the adaptive immune response, which includes cells and molecules with antigen specificity, such as T-cells and the antibody producing B-cells. B-cells cannot mature into antibody producing cells without help from T-cells of the corresponding specificity. T-cells that participate in the stimulation of allergic immune responses are primarily of the Th2 type. Establishment of a new balance between Th1 and Th2 cells has been proposed to be beneficial and central to the immunological mechanism of specific allergy vaccination. Whether this is brought about by a reduction in Th2 cells, a shift from Th2 to Th1 cells, or an up-regulation of Th1 cells is controversial. Recently, regulatory T-cells have been proposed to be important for the mechanism of allergy vaccination. According to this model regulatory T-cells, i.e. Th3 or Tr1 cells, down-regulate both Th1 and Th2 cells of the corresponding antigen specificity. In spite of these ambiguities it is generally believed that an active vaccine must have the capacity to stimulate allergen specific T-cells, preferably TH1 cells.

Specific allergy vaccination (SAV), formerly known as Specific Immunotheraphy or Hyposensitization, has been used for the treatment of Type 1 IgE mediated allergic disease since the beginning of this century.

The general benefits obtained through SAV are: a) reduction of allergic symptoms and medicine consumption, b) improved tolerance towards the allergens in the eyes, nose and lungs and c) reduced skin reactivity (early and late phase reactions).

The basic mechanism behind the improvement obtained by SAV is unknown, but a number of common features can be extracted from the numerous SAV studies performed in the last decades: 1) the amount of total IgE is unchanged during the treatment period, 2) the amount of allergen specific IgE increases transiently during updosing, then it falls back to the initial (pretreatment) level, 3) the epitope specificity and affinity of IgE remains unchanged, 4) allergen specific IgG, in particularly IgG4, raises sharply during SAV, 5) a new Th0/1/Reg response is apparently initiated and 6) the Th2 response seem unchanged. There is no correlation between the effect induced by SAV and the onset of specific IgG.

SAV induces a new immune response which matures during the treatment period (Th0/1 T-cells are recruited, an allergen specific IgX (X may be A1, A2, G1, G2, G3, G4, M or D) is initiated). As the affinity (or amount/affinity) of the new antibody response, IgX, has matured, IgX may compete efficiently with IgE for the allergen(s), inhibiting the "normal" Th2 based allergic response characterised by the cross-linking of receptor bound IgE on the surface of mast-cells and basophils. Hence, clinical symptoms will gradually be reduced.

Specific allergy vaccination is, in spite of its virtues, not in widespread use, primarily for two reasons. One reason is the inconveniences associated with the traditional vaccination programme that comprises repeated vaccinations i.a. injections over a several months. The other reason is, more importantly, the risk of allergic side reactions. Ordinary vaccinations against infectious agents are efficiently performed using a single or a few high dose immunizations. This strategy, however, cannot be used for allergy vaccination since a pathological immune response is already ongoing.

Conventional specific allergy vaccination is therefore carried out using multiple subcutaneous immunizations applied over an extended time period. The course is divided in two phases, the up dosing and the maintenance phase. In the up dosing phase increasing doses are applied, typically over a 16-week period, starting with minute doses. When the recommended maintenance dose is reached, this dose is applied for the maintenance phase, typically with injections every six weeks. Following each injection the patient must remain under medical attendance for 30 minutes due to the risk of anaphylactic side reactions, which in principle although extremely rare could be life-threatening. In addition, the clinic should be equipped to support emergency treatment. There is no doubt that a vaccine based on a different route of administration would eliminate or reduce the risk for allergic side reactions inherent in the current subcutaneous based vaccine as well as would facilitate a more widespread use, possibly even enabling self vaccination at home.

Attempts to improve vaccines for specific allergy vaccination have been performed for over 30 years and include multifarious approaches. Several approaches have addressed the allergen itself through modification of the IgE reactivity,

Allergen extracts purified from grass pollen, tree pollen and house dust mites have been used as pharmaceuticals for many years without any acknowledged problems with toxicity.

### SUMMARY OF THE INVENTION

In a first aspect of the invention, the present invention relates to a method of developing a process for producing an allergen extract from a biological allergen source material comprising the steps of
a) subjecting the source material to a first extraction and purification process to produce a first purified allergen extract,
b) subjecting the first purified allergen extract to one or more toxicity tests to examine the first purified allergen extract for a potential content of one or more toxic components,
c) if the toxicity test shows that the toxic component has not been removed, subjecting the allergen extract to a first modified extraction and purification method to produce a first modified purified allergen extract,
d) subjecting the first modified purified allergen extract to one or more toxicity tests to examine the first modified purified allergen extract for a potential content of one or more toxic components,
e) repeating step c) to d) until the toxic component has been removed to identify an extraction and purification method, which removes the potential toxic component.

Recent research has surprisingly shown that some types of allergen extracts from biological allergen source materials, e.g. extracts from grass pollen, which has hitherto been believed to contain no toxic components, in fact may contain components, which in at least some toxicity tests results in the tests being positive. The present invention is based on the recognition of this technical problem. This finding is surprising, since such extracts have been used for many years without any knowledge of or experimental findings to this effect. Also, it is surprising, since the biological source materials, from which the extracts are obtained, are biological materials, which in general are believed to be harmless and non-toxic by nature, and for example also serves as feed for various animals.

The present invention is further based on the recognition that the identification of the toxic component(s) of such allergen extracts is a very challenging and time-consuming task because of the complexity of the composition of the allergen extract, and that therefore this approach for eliminating toxic components is undesirable. On the basis of this recognition, the present invention is based on the further recognition that the technical problem may instead be solved by application of an empirical trial and error method comprising the steps of removing a smaller or larger fraction of the allergen extract suspected to contain the toxic component(s) using a suitable purification method, and examining whether the removed fraction in fact does contain the toxic component(s) by testing the resulting purified allergen extract and/or the removed fraction thereof for its toxicity in one or more suitable toxicity tests.

The present method of developing a process for producing an allergen extract from a biological allergen source material may e.g. be used in a situation, wherein a toxic component is observed in a purified allergen extract resulting from an existing production process already in use. Also, the method of the invention may e.g. be used in a situation, wherein an existing production process is changed for a reason unrelated to the toxicity problem for validating the new production process for its ability to remove toxic components of the crude allergen extract.

In a second aspect of the invention, the present invention further relates to a method of developing a process for producing an allergen composition from a gene expression medium mixture comprising the steps of
h) subjecting the medium mixture to a first purification process to produce a first purified allergen composition,
i) subjecting the first purified allergen composition to one or more toxicity tests to examine the first purified allergen composition for a potential content of one or more toxic components,
j) if the toxicity test shows that the toxic component has not been removed, subjecting the allergen extract to a first modified purification method to produce a first modified purified allergen composition,
k) subjecting the first modified purified allergen composition to one or more toxicity tests to examine the first modified purified allergen composition for a potential content of one or more toxic components,
I) repeating step j) to k) until the toxic component has been removed to identify a purification method, which removes the potential toxic component.

The second aspect of the invention is based on the recognition that when developing a process for producing an allergen protein by recombinant methods, the resulting allergen composition resulting from the purification of the allergen from the gene expression medium mixture may contain a toxic component, and that such an allergen composition may be screened for the presence of such a toxic component in the same manner as an allergen extract purified from a biological allergen source material.

In a third aspect of the invention, the present invention relates to a method of evaluating a process for producing an allergen extract from a biological allergen source material for its ability to remove toxic components of the allergen extract, the method comprising the steps of
s) subjecting the source material to an extraction and purification process to produce a purified allergen extract,
t) subjecting the purified allergen extract to one or more toxicity tests to examine the purified allergen extract for a potential content of one or more toxic components, and
u) evaluating the process for its ability to remove toxic components of the allergen extract on the basis of the results of the toxicity test.

The third aspect of the invention is based on the same recognitions as the first aspect of the invention. The method of the fourth aspect of the invention may be used to examine a process with respect to its ability to remove potential toxic components of the allergen extract.

In a fourth aspect of the invention, the present invention relates to a method of evaluating an allergen extract from a biological allergen source material for its content of toxic components, the method comprising the steps of
x) subjecting the source material to an extraction and purification process to produce a purified allergen extract,
y) subjecting the purified allergen extract to one or more toxicity tests to examine the purified allergen extract for a potential content of one or more toxic components, and
z) evaluating the allergen extract for its content of toxic components on the basis of the results of the toxicity test.

The fourth aspect of the invention is based on the same recognitions as the first aspect of the invention. The method of the fourth aspect of the invention may be used to examine a biological allergen source material for its content of toxic components.

In a fifth aspect of the invention the present invention relates to a method of evaluating a biological allergen source material for its content of toxic components, the method comprising the steps of
x') subjecting the source material to an extraction process to produce an allergen extract,
y') subjecting the allergen extract to one or more toxicity tests to examine the allergen extract for a potential content of one or more toxic components, and
z') evaluating the source material for its content of toxic components on the basis of the results of the toxicity test.

The fifth aspect of the invention is based on the same recognitions as the first aspect of the invention. The method of the fifth aspect of the invention may be used to examine a biological allergen source material for its content of toxic components.

In a sixth aspect of the invention, the present invention relates to a method of evaluating a gene expression medium mixture for its content of toxic components, the method comprising the steps of
y") subjecting the medium mixture to one or more toxicity tests to examine the allergen extract for a potential content of one or more toxic components, and
z") evaluating the medium mixture for its content of toxic components on the basis of the results of the toxicity test.

### DETAILED DESCRIPTION OF THE INVENTION

### Allergen extract

The allergen extract according to the present invention may be an extract of a biological allergen source material containing any naturally occurring protein allergen that has been reported to induce allergic, i.e. IgE mediated, reactions upon their repeated exposure to an individual. Examples of naturally occurring allergens include pollen allergens (tree-, herb, weed-, and grass pollen allergens), insect allergens (inhalant, saliva and venom allergens, e.g. mite allergens, cockroach and midges allergens, hymenopthera venom allergens), animal hair and dandruff allergens (from e.g. dog, cat, horse, rat, mouse etc.), and food allergens. Important pollen allergens from trees, grasses and herbs are such originating from the taxonomic orders of Fagales, Oleales, Pinales and platanaceae including i.a. birch (Betula), alder (Alnus), hazel (Corylus), hornbeam (Carpinus) and olive (Olea), cedar (Cryptomeria and Juniperus), Plane tree (Platanus), the order of Poales including i.a. grasses of the genera Lolium, Phleum, Poa, Cynodon, Dactylis, Holcus, Phalaris, Secale, and Sorghum, the orders of Asterales and Urticales including i.a. herbs of the genera Ambrosia, Artemisia, and Parietaria. Other important inhalation allergens are those from house dust mites of the genus Dermatophagoides and Euroglyphus, storage mite e.g Lepidoglyphys, Glycyphagus and Tyrophagus, those from cockroaches, midges and fleas e.g. Blatella, Periplaneta, Chironomus and Ctenocepphalides, and those from mammals such as cat, dog and horse, venom allergens including such originating from stinging or biting insects such as those from the taxonomic order of Hymenoptera including bees (superfamily Apidae), wasps (superfamily Vespidea), and ants (superfamily Formicoidae). Important inhalation allergens from fungi and moulds are i.a. such originating from the genera Alternaria and Cladosporium.

In a particular embodiment of the invention the allergen is selected from the group consisting of Bet v 1, Aln g 1, Cor a 1, Car b 1, Que a 1, Cry j 1, Cry j 2, Cup a 1, Cup s 1, Jun a 1, Jun a 2, Jun a 3, Ole e 1, Lig v 1, Pla l 1, Pla a 2, Amb a 1, Amb a 2, Amb t 5, Art v 1, Art v 2 Par j 1, Par j 2, Par j 3, Sal k 1, Ave e 1, Cyn d 1, Cyn d 7, Dac g 1, Fes p 1, Hol l1, Lol p 1 and 5, Pha a 1, Pas n 1, Phl p 1, Phl p 5, Phl p 6, Poa p 1, Poa p 5, Sec c 1, Sec c 5, Sor h 1, Der f 1, Der f 2, Der p 1, Der p 2, , Der p 7, Der m 1, Eur m 2, Gly d 1, Lep d 2, Blo t 1, Tyr p 2, Bla g 1, Bla g 2, Per a 1, Fel d 1, Can f 1, Can f 2 , Bos d 2, Equ c 1, Equ c 2, Equ c 3 , Mus m 1, Rat n 1, Apis m 1, Api m 2 , Ves v 1, Ves v 2, Ves v 5, Dol m 1, Dil m 2, Dol m 5, Pol a 1, Pol a 2, Pol a 5, Sol i 1, Sol i 2, Sol i 3 and Sol i 4, Alt a 1, Cla h 1, Asp f 1, Bos d 4, Mal d 1, Gly m 1, Gly m 2, Gly m 3, Ara h 1, Ara h 2, Ara h 3, Ara h 4 and Ara h 5.

In a preferred embodiment of the invention the allergen is selected from the group consisting of a tree pollen allergen, a grass pollen allergen, a house dust mite allergen, a storage mite allergen, a weed allergen, a mould allergen, a cat allergen and a dog allergen.

### Toxicity test

The toxicity test used in the method of the invention may be any conventional toxicity test, which is relevant for testing an active substance in the form of an allergen extract, including pre-clinical tests and clinical tests and endotoxin tests.

Conventional toxicity tests include i.a. any test approved by ICH (International Conference on Harmonisation of technical requirements for registration of pharmaceutical for human use). Pre-clinical toxicity tests include in vivo and in vitro tests. Categories of pre-clinical toxicity tests include (referring to ICH itemisation) S1) Carcinogenicity Studies, S2) Genotoxicity Studies, S3) Toxicokinetics and Pharmacokinetics, S4) Toxicity Testing, S5) Reproductive Toxicology, S6) Biotechnology Products, S7) Pharmacology Studies, S8) Immunotoxicology Studies, and M3) Joint Safety/Efficacy Topic. Reference is made to the ICH guidelines as in force on the filing date of this patent application. The following is an edited extract of the ICH guidelines.

### S1 Carcinogenicity Studies

The basic scheme of Carcinogenicity Studies comprises one long-term rodent carcinogenicity study, plus one other study of a type that supplements the long term carcinogenicity study and provides additional information that is not readily available from the long term assay.

The species for a long-term carcinogenicity study should be selected based on considerations that include the following: (a) Pharmacology, (b) Repeated-dose toxicology, (c) Metabolism, (d) Toxicokinetics and (e) Route of administration (e.g., less common routes such as dermal and inhalation). In the absence of clear evidence favouring one species, it is recommended that the rat be selected.

Additional in vivo tests for carcinogenicity may be either (a) a short or medium-term in vivo rodent test systems or (b) a long-term carcinogenicity study in a second rodent species.

Re (a): Possibilities should focus on the use of in vivo models providing insight into carcinogenic endpoints. These may include models of initiation-promotion in rodents, or models of carcinogenesis using transgenic or neonatal rodents. Considerations in the choice of short or medium term tests for carcinogenicity are as follows: Emphasis should be placed on selection of a test method that can contribute information valuable to the overall "weight of evidence" for the assessment of carcinogenic potential. The rationale for this choice should be documented and based on information available at the time of method selection about the pharmaceutical such as pharmacodynamics and exposure compared to human or any other information that may be relevant. This rationale should include a scientific discussion of the strengths and weaknesses of the method selected for the pharmaceutical.

Mechanistic studies are often useful for the interpretation of tumour findings in a carcinogenicity study and can provide a perspective on their relevance to human risk assessment. The need for or the design of an investigative study will be dictated by the particular properties of the drug and/or the specific results from the carcinogenicity testing. Dose dependency and the relationship to carcinogenicity study conditions should be evaluated in these investigational studies. Suggestions include:

### Cellular changes

Relevant tissues may be examined for changes at the cellular level using morphological, histochemical, or functional criteria. As appropriate, attention may be directed to such changes as the dose-relationships for apoptosis, cell proliferation, liver foci of cellular alteration, or changes in intercellular communication.

### Biochemical measurements

Depending on the putative mode of tumorigenic action, investigations could involve measurements of:
- plasma hormone levels, e.g. T3/T4, TSH, prolactin
- growth factors
- binding to proteins such as α2µ-globulin
- tissue enzyme activity, etc.
In some situations, it may be possible to test a hypothesis of, for example, a hormone imbalance with another study in which the imbalance has been, at least in part, compensated.

### Considerations for additional genotoxicity testing

Additional genotoxicity testing in appropriate models may be invoked for compounds that were negative in the standard test battery but which have shown effects in a carcinogenicity test with no clear evidence for an epigenetic mechanism. Additional testing can include modified conditions for metabolic activation in in vitro tests or can include in vivo tests measuring genotoxic damage in target organs of tumor induction (e.g., DNA damage and repair tests, 32P-postlabeling, mutation induction in transgenes).

### Modified protocols

Modified protocols may be helpful to clarify the mode of tumorigenic action of the test substance. Such protocols might include groups of animals to explore, for example, the consequence of interrupted dosage regimens, or the reversibility of cellular changes after cessation of dosing.

### S2 Genotoxicity Studies

Genotoxicity Studies include i) a test for gene mutation in bacteria, (ii) an in vitro test with cytogenetic evaluation of chromosomal damage with mammalian cells, (iii) an in vitro mouse lymphoma tk assay, and (iv) an in vivo test for chromosomal damage using rodent hematopoietic cells.

Re i): It is appropriate to assess genotoxicity in a bacterial reverse mutation test, e.g. an Ames test. This test has been shown to detect relevant genetic changes and the majority of genotoxic rodent carcinogens. Current guidelines for the detection of bacterial mutagens employ several strains to detect base substitution and frameshift point mutations. The Salmonella typhimurium strains mentioned in guidelines (normally TA1535, TA1537, TA98 and TA100) will detect such changes at G-C (guanine-cytosine) sites within target histidine genes. It is clear from the literature that some mutagenic carcinogens also modify A-T (adenine-thymine) base pairs. Therefore the standard set of strains used in bacterial mutation assays should include strains that will detect point mutations at A-T sites, such as Salmonella typhimurium TA102, which detects such mutations within multiple copies of hisG genes or Escherichia coli WP2 uvrA, which detects these mutations in the trpE gene or the same strain possessing the plasmid (pKM101), which carries mucAB genes that enhance error prone repair. In conclusion, the following base set of bacterial strains should be used for routine testing: the strains cited below are all Salmonella typhimurium isolates, unless specified otherwise.
1. TA98; 2. TA100; 3. TA1535; 4. TA1537 or TA97 or TA97a; 5. TA102 or Escherichia coli WP2 uvrA or Escherichia coli WP2 uvrA (pKM101). In order to detect cross-linking agents it may be preferable to select Salmonella typhimurium TA 102 or to add a repair proficient Escherichia coli strain, such as WP2 pKM101. It is noted that such compounds are detected in assays that measure chromosome damage.

Re ii): DNA damage considered to be relevant for mammalian cells and not adequately measured in bacteria should be evaluated in mammalian cells. Several mammalian cell systems are in use: systems that detect gross chromosomal damage (in vitro tests for structural and numerical chromosomal aberrations), systems that detect primarily gene mutations, and a system that detects gene mutations and clastogenic effects (mouse lymphoma tk assay). With appropriate test protocols the various in vitro tests for chromosomal damage and the mouse lymphoma tk assay yield results with a high level of congruence for compounds that are regarded as genotoxic but yield negative results in the bacterial reverse mutation assay. Therefore, these systems are currently considered interchangeable when used together with other genotoxicity tests in a standard battery for genotoxicity testing of pharmaceuticals, if these test protocols are used.

Re iii): An in vivo test for genetic damage should usually be a part of the test battery to provide a test model in which additional relevant factors (absorption, distribution metabolism, excretion) that may influence the genotoxic activity of a compound are included. As a result, in vivo tests permit the detection of some additional genotoxic agents. An in vivo test for chromosomal damage in rodent hematopoietic cells fulfills this need. This in vivo test for chromosomal damage in rodents could be either an analysis of chromosomal aberrations in bone marrow cells or an analysis of micronuclei in bone marrow or peripheral blood erythrocytes. Tests measuring chromosomal aberrations in nucleated bone marrow cells in rodents can detect a wide spectrum of changes in chromosomal integrity. These changes almost all result from breakage of one or more chromatids as the initial event. Breakage of chromatids or chromosomes can result in micronucleus formation if an acentric fragment is produced; therefore assays detecting either chromosomal aberrations or micronuclei are acceptable for detecting clastogens. Micronuclei can also result from lagging of one or more whole chromosome(s) at anaphase and thus micronucleus tests have the potential to detect some aneuploidy inducers. In conclusion either the analysis of chromosomal aberrations in bone marrow cells or the measurement of micronucleated polychromatic erythrocytes in bone marrow cells in vivo is acceptable for the detection of clastogens. The measurement of micronucleated immature (e.g., polychromatic) erythrocytes in peripheral blood is an acceptable alternative in the mouse, or in any other species in which the inability of the spleen to remove micronucleated erythrocytes has been demonstrated, or which has shown an adequate sensitivity to detect clastogens/aneuploidy inducers in peripheral blood. Extensive studies of the activity of known clastogens in the mouse bone marrow micronucleus test have shown that in general male mice are more sensitive than female mice for micronucleus induction. Quantitative differences in micronucleus induction have been identified between the sexes, but no qualitative differences have been described. Where marked quantitative differences exist, there is invariably a difference in toxicity between the sexes. If there is a clear qualitative difference in metabolites between male and female rodents, then both sexes should be used. Similar principles can be applied for other established in vivo tests. Both rats and mice are deemed acceptable for use in the bone marrow micronucleus test. In summary, unless there are obvious differences in toxicity or metabolism between male and female rodents, then males alone are sufficient for use in bone marrow micronucleus tests. If gender-specific drugs are to be tested, then normally animals of the corresponding sex should be used.

### S3 Toxicokinetics and Pharmacokinetics

The primary objective of toxicokinetics is to describe the systemic exposure achieved in animals and its relationship to dose level and the time course of the toxicity study. Secondary objectives are i) to relate the exposure achieved in toxicity studies to toxicological findings and contribute to the assessment of the relevance of these findings to clinical safety, ii) to support the choice of species and treatment regimen in non-clinical toxicity studies, and iii) to provide information which, in conjunction with the toxicity findings, contributes to the design of subsequent non-clinical toxicity studies.

These objectives may be achieved by the derivation of one or more pharmacokinetic parameters from measurements made at appropriate time points during the course of the individual studies. These measurements usually consist of plasma (or whole blood or serum) concentrations for the parent compound and/or metabolite(s) and should be selected on a case-by-case basis. Plasma (or whole blood or serum) AUC, Cmax and C(time) are the most commonly used parameters in assessing exposure in toxicokinetics studies. For some compounds it will be more appropriate to calculate exposure based on the (plasma protein) unbound concentration. These data may be obtained from all animals on a toxicity study, in representative subgroups, in satellite groups or in separate studies.

Toxicity studies which may be usefully supported by toxicokinetic information include single and repeated-dose toxicity studies, reproductive, genotoxicity and carcinogenicity studies. Toxicokinetic information may also be of value in assessing the implications of a proposed change in the clinical route of administration.

A comprehensive knowledge of the absorption, distribution, metabolism and elimination of a compound is important for the interpretation of pharmacology and toxicology studies. Tissue distribution studies are essential in providing information on distribution and accumulation of the compound and/or metabolites, especially in relation to potential sites of action; this information may be useful for designing toxicology and pharmacology studies and for interpreting the results of these experiments. Tissue distribution studies are an important component in the non-clinical kinetics programme. For most compounds, it is expected that single dose tissue distribution studies with sufficient sensitivity and specificity will provide an adequate assessment of tissue distribution and the potential for accumulation. Thus, repeated dose tissue distribution studies should not be required uniformly for all compounds and should only be conducted when appropriate data cannot be derived from other sources. Repeated dose studies may be appropriate under certain circumstances based on the data from single dose tissue distribution studies, toxicity and toxicokinetic studies. The studies may be most appropriate for compounds which have an apparently long half life, incomplete elimination or unanticipated organ toxicity. The design and timing of repeated dose tissue distribution studies should be determined on a case-by-case basis.

### Endotoxin test

Conventional endotoxin tests include the Limulus Amoebucyte Lysate (LAL) in vitro test and the rabbit fever in vivo test.

### Extraction and purification process

### Basic steps of an extraction and purification process

The steps of a process for extracting and purifying an allergen extract from a biological allergen source material depend on the type of the source material, as the nature of various source materials, such as grass pollen, tree pollen and house dust mite residues, differs significantly. Typical steps include an extraction step, a separation step to remove the solid residues of the source material, and purification of the allergen extract.

The liquid extraction agent may e.g. be water or an aqueous buffer. The extraction step is carried out by mixing a liquid extraction agent with a biological allergen source material to form a suspension of the solid phase source material in the liquid phase of extraction agent, and the mixture is agitated. The extraction is preferably continued for a selected period of time of e.g. from 1 to 24 hours, preferably from 2 to 12 hours, during which the temperature is adjusted to e.g. from 2 to 25 °C, preferably from 3 to 15 °C, and the pH is adjusted to from 5 to 9, preferably from 6 to 8. At the end of the extraction period an allergen extract mixture is obtained, which comprises a solid phase composed of the source material residues, i.e. the solid matter remaining from the extraction, and a liquid phase containing the molecules extracted from the source material.

The allergen extract mixture is then subjected to a separation step, wherein at least most of the source material residues, in particular the larger particles thereof, is removed. This separation may be carried out e.g. by centrifugation or filtration. The removal of the source material residues results in a crude allergen extract, which contains the extracted molecules in solution and to a certain extent smaller particles, depending on the separation method and equipment used.

Optionally, the crude allergen extract may then be concentrated e.g. by ultrafiltration, precipitation and/or ion exchange chromatography.

The crude allergen extract may then be subjected to one or more purification steps to remove a particular fraction of the allergen extract, cf. the section below.

Finally, the purified allergen extract may then be concentrated once more e.g. by ultrafiltration, precipitation and/or ion exchange chromatography.

### Purification steps for removing a particular fraction of an allergen extract

Once one or more toxic components has been identified in the purified allergen extract resulting from the first extraction and purification process, a modified extraction and purification process should be designed and selected with an object of attempting to remove the toxic component. The modified process may include any conventional purification step to achieve this object. If nothing is known or suggested about the identity of the toxic component, the sequence of purification steps of the modified extraction and purification method may be selected at random and tested one sequence after another as a trial and error approach. The modified process may be designed taking the first process, which did not remove the toxic component, as a starting point, and modifying the first process by replacing one purification step with a different purification step or by adding an additional purification step.

The selection of the purification step or steps of the modified process may be selected on the basis of the fact that the purification step is simple in use, cost effective etc. Also, the purification step may be selected based on physical properties of the molecules, which are to be removed. Physical properties commonly used as a basis for separation are molecular size, ionic charge, specific sequences and structures of the molecule and hydrophobic regions of the molecule.

Suitable purification steps may e.g. be selected from the group consisting of filtration, centrifugation, ultrafiltration, diafiltration, membrane filtration, dialysis, salting-out, precipitation, gel electrophoresis and column chromatography, including gel filtration, ion exchange chromatography, affinity chromatography and hydrophobic interaction chromatography.

In a particular embodiment of the invention, the allergen extract is subjected to a molecular size fraction removal step, which may be carried out e.g. by means of ultrafiltration, diafiltration, dialysis and/or gel filtration. In the molecular size fraction removal step, molecules having a selected range of molecular size, e.g. a low molecular or a high molecular fraction, are removed.

### Method of developing a process for producing an allergen composition from a gene expression medium mixture

The comments given in relation to the method of developing a process for producing an allergen extract from a biological allergen source material also apply to the method of developing a process for producing an allergen composition from a gene expression medium mixture.

The gene expression medium mixture is the mixture resulting from a fermentation process of expressing a given protein allergen by recombinant methods involving cultivating host cell microorganisms, such as bacteria and fungi, in a suitable growth medium. The protein allergen expressed may partly or fully be contained as an intracellular protein in the host cell. In this situation the process for producing an allergen composition from a gene expression medium mixture may include a step of degrading the cell. Such a cell degradation step may be carried out by mechanical force, high pressure homogenisation, ultrasound (sonification), chemical means and enzymatic means.

### A method of evaluating a biological allergen source material for its content of toxic components

As mentioned above the method according to the fifth aspect of the invention may be used in itself to examine a biological allergen source material for its content of toxic components. Also, this aspect of the method of the invention may be used in conjunction with any of the other aspects of the invention to examine, whether an allergen extract of a source material contains any toxic components prior to purification.

### DEFINITIONS

In connection with the present invention, the following terms are defined as follows:

The term "ultrafiltration" means up-concentration of a solution in a membrane filter, i.e. the permeate of the solution is discarded and the retentate of the solution may be used or recycled and subjected to another filtration. The recycling may be continued until a desired concentration of the solution is achieved.

The term "diafiltration" means filtration of a solution in a membrane filter while maintaining the same volume of the solution, i.e. the permeate of the solution is discarded and a volume of solvent, e.g. water, is added to the retentate of the solution, which may be used or recycled and subjected to another filtration. This may be continued until the desired concentration of the solution is achieved.

The term "dialysis" means removal of dissolved components by osmosis from a solution, e.g. by filling the solution into perforated tubes, which are then placed in a container filled with solvent and left to stand for a period of time, during which the dissolved components by osmosis diffuse out of the tubes into the surrounding solvent.

The expression "toxic component" means any component, which is positive in any conventional toxicity test, including those mentioned by ICH (International Conference on Harmonisation of technical requirements for registration of pharmaceutical for human use) on the date of filing of this application, or an endotoxin test.

The expression "endotoxin test" means any conventional endotoxin test.

The term "endotoxin" means a poisonous substance present in or originating from a microorganism.

## Claims

1. A method of developing a process for producing an allergen extract from a biological allergen source material comprising the steps of
a) subjecting the source material to a first extraction and purification process to produce a first purified allergen extract,
b) subjecting the first purified allergen extract to one or more toxicity tests to examine the first purified allergen extract for a potential content of one or more toxic components,
c) if the toxicity test shows that the toxic component has not been removed, subjecting the allergen extract to a first modified extraction and purification method to produce a first modified purified allergen extract,
d) subjecting the first modified purified allergen extract to one or more toxicity tests to examine the first modified purified allergen extract for a potential content of one or more toxic components,
e) repeating step c) to d) until the toxic component has been removed to identify an extraction and purification method, which removes the potential toxic component.

2. A method according to claim 1, wherein the first modified extraction and purification process comprises a step, which removes a fraction of the allergen components having a particular molecular size.

3. A method according to claim 2, wherein the first modified extraction and purification process comprises a step, which removes a low molecular fraction of the allergen components.

4. A method according to claim 2, wherein the first modified extraction and purification process comprises a step, which removes a high molecular fraction of the allergen components.

5. A method according to any of claims 2-4, wherein the molecular size fraction removal step is carried out by means of ultrafiltration, diafiltration, dialysis or a gel filtration.

6. A method according to any of claims 1-5, wherein the toxicity test is a genotoxicity test selected from the group consisting of i) a test for gene mutation in bacteria, (ii) an in vitro test with cytogenetic evaluation of chromosomal damage with mammalian cells, (iii) an in vitro mouse lymphoma tk assay, and (iv) an in vivo test for chromosomal damage using rodent hematopoietic cells.

7. A method according to any of the preceding claims, wherein the biological allergen source material is grass pollen.

8. A method of developing a process for producing an allergen composition from a gene expression medium mixture comprising the steps of
h) subjecting the medium mixture to a first purification process to produce a first purified allergen composition,
i) subjecting the first purified allergen composition to one or more toxicity tests to examine the first purified allergen composition for a potential content of one or more toxic components,
j) if the toxicity test shows that the toxic component has not been removed, subjecting the allergen extract to a first modified purification method to produce a first modified purified allergen composition,
k) subjecting the first modified purified allergen composition to one or more toxicity tests to examine the first modified purified allergen composition for a potential content of one or more toxic components,
l) repeating step j) to k) until the toxic component has been removed to identify a purification method, which removes the potential toxic component.

9. A method of evaluating a process for producing an allergen extract from a biological allergen source material for its ability to remove toxic components of the allergen extract, the method comprising the steps of
s) subjecting the source material to an extraction and purification process to produce a purified allergen extract,
t) subjecting the purified allergen extract to one or more toxicity tests to examine the purified allergen extract for a potential content of one or more toxic components, and
u) evaluating the process for its ability to remove toxic components of the allergen extract on the basis of the results of the toxicity test.

10. A method of evaluating an allergen extract from a biological allergen source material for its content of toxic components, the method comprising the steps of
x) subjecting the source material to an extraction and purification process to produce a purified allergen extract,
y) subjecting the purified allergen extract to one or more toxicity tests to examine the purified allergen extract for a potential content of one or more toxic components, and
z) evaluating the allergen extract for its content of toxic components on the basis of the results of the toxicity test.

11. A method of evaluating a biological allergen source material for its content of toxic components, the method comprising the steps of
x') subjecting the source material to an extraction process to produce an allergen extract,
y') subjecting the allergen extract to one or more toxicity tests to examine the allergen extract for a potential content of one or more toxic components, and
z') evaluating the source material for its content of toxic components on the basis of the results of the toxicity test.

12. A method of evaluating a gene expression medium mixture for its content of toxic components, the method comprising the steps of
y") subjecting the medium mixture to one or more toxicity tests to examine the allergen extract for a potential content of one or more toxic components, and
z") evaluating the medium mixture for its content of toxic components on the basis of the results of the toxicity test.
